# FASCICULE DE BREVET EUROPEEN

(11) **EP 1 377 263 B1**
(45) Date de publication et mention de la délivrance du brevet: **17.02.2010**
(21) Numéro de dépôt: 02724399.7
(22) Date de dépôt: 02.04.2002
(51) Int. Cl.: A61Q 5/10, C09B 44/16, C09B 44/12, A61K 8/49, A61K 8/41

(54) **COMPOSITION TINCTORIALE POUR LA TEINTURE DES FIBRES KERATINIQUES COMPRENANT UN COLORANT DIAZOIQUE DICATIONIQUE PARTICULIER**
EIN DIKATIONISCHER DIAZOFARBSTOFF ENTHALTENDE FARBSTOFFZUSAMMENSETZUNG ZUM FÄRBEN VON KERATINISCHEN FASERN
NOVEL DYEING COMPOSITION FOR DYEING KERATINOUS FIBRES COMPRISING A PARTICULAR DICATIONIC DIAZO DYE

(30) Priorité: 02.04.2001 FR 0104469
(43) Date de publication de la demande: 07.01.2004
(73) Titulaire: L'Oréal, 75008 Paris (FR)
(72) Inventeur: VIDAL, Laurent, F-75013 Paris (FR); LEDUC, Madeleine Résidence Les Chèvrefeuilles, F-75011 Paris (FR)
(74) Mandataire: Wattremez, Catherine
(86) Numéro de dépôt international: PCT/FR2002/001137
(87) Numéro de publication internationale: WO 2002/078596

(56) Documents cités:
- EP-A- 0 714 954
- EP-A- 0 962 219
- FR-A- 1 584 965
- FR-A- 2 741 798
- US-A- 3 271 383
- US-A- 4 557 732
- PATENT ABSTRACTS OF JAPAN vol. 004, no. 050 (C-007), 16 avril 1980 (1980-04-16) & JP 55 022638 A (YAMAGUCHI HARUO), 18 février 1980 (1980-02-18) cité dans la demande
- DATABASE CA [en ligne] CHEMICAL ABSTRACTS SERVICE, COLUMBUS, OHIO, US; MANABE, OSAMU ET AL: "Basic azo dyes for polyacrylonitrile fibers" retrieved from STN Database accession no. 72:134134 XP002188108 & JP 45 004332 B (ASAHI DYESTUFFS MANUFG. CO., LTD.) 13 février 1970 (1970-02-13)
- CHEMICAL ABSTRACTS, vol. 70, no. 4, 27 janvier 1969 (1969-01-27) Columbus, Ohio, US; abstract no. 12645x, KIPRIANIV ET AL: "Cyanine dyes with two conjugated chromophores" page 63; XP002188107 & UKR. KHIM. ZH., vol. 34, no. 8, 1968, pages 795-799,

## Description

L'invention a pour objet une nouvelle composition tinctoriale pour la teinture des fibres kératiniques, en particulier des cheveux humains, contenant un colorant diazoïque dicationique particulier, ainsi que le procédé de teinture des fibres kératiniques mettant en oeuvre une telle composition. L'invention a aussi pour objet des colorants dicationiques diazoïques nouveaux.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

Ce procédé de coloration d'oxydation consiste à appliquer sur le fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres.

Il est connu par exemple d'utiliser des colorants directs du type nitrés benzéniques, anthraquinoniques, nitropyridiniques, des colorants du type azoïques, xanthéniques, acridiniques aziniques ou des colorants triarylméthane.

Les colorations qui en résultent sont des colorations particulièrement chromatique qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.

Il est connu d'utiliser des colorants directs en combinaison avec des agents oxydants. Cependant, les colorants directs sont généralement sensibles à faction des agents oxydants tels que l'eau oxygénée, et les agents réducteurs tels que le bisulfite de sodium, ce qui les rendent généralement difficilement utilisables dans les compositions de teinture directe éclaircissantes à base d'eau oxygénée et à base d'un agent alcalinisant ou dans des compositions de teinture d'oxydation en association avec des précurseurs du type bases d'oxydation ou coupleurs.

Par exemple, il a été proposé dans les demandes de brevets FR-1 584 965 et JP-062 711 435 de teindre les cheveux avec des compositions de teinture à base de colorants directs nitrés et/ou de colorants dispersés azoïques et d'eau oxygénée ammoniacale en appliquant sur les cheveux un mélange desdits colorants et dudit oxydant, réalisé juste avant l'emploi. Mais les colorations obtenues se sont révélées insuffisamment tenaces et disparaissent aux shampooings en laissant apparaître l'éclaircissement de la fibre capillaire. Une telle coloration devient inesthétique en évoluant au cours du temps.

On a également proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pause des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

Plus récemment, la demande de brevet FR 2 741 798 a décrit des compositions tinctoriales contenant des colorants directs comportant au moins un atome d'azote quatemisé du type azoïque ou azométhine, lesdites compositions étant à mélanger extemporanément à pH basique à une composition oxydante. Ces compositions permettent d'obtenir des colorations avec des reflets homogènes, tenaces et brillants. Cependant, elles ne permettent pas de teindre les fibres kératiniques avec autant de puissance qu'avec des compostions de coloration d'oxydation.

Il existe donc un réel besoin de rechercher des colorants directs chromatiques qui permettent de teindre les fibres kératiniques aussi puissamment que les colorants d'oxydation, qui soient aussi stables qu'eux à la lumière, soient également résistants aux intempéries, aux lavages et à la transpiration, et en outre, suffisamment stables en présence d'agents oxydants et réducteurs pour pouvoir obtenir simultanément un éclaircissement de la fibre soit par utilisation de compositions directes éclaircissantes les contenant, soit par l'utilisation de compositions de coloration d'oxydation les contenant. Il existe aussi un réel besoin de rechercher des colorants directs qui permettent de teindre les fibres kératiniques pour obtenir une gamme très large de couleurs, en particulier très chromatiques, sans oublier les nuances dites « fondamentale » comme les noirs et les marrons.

Ces buts sont atteints avec la présente invention qui a pour objet une composition pour la teinture des fibres kératiniques et en particulier des fibres kératiniques humaines telles que les cheveux, comprenant au moins un colorant diazoïque dicationique de formule (I) suivante : 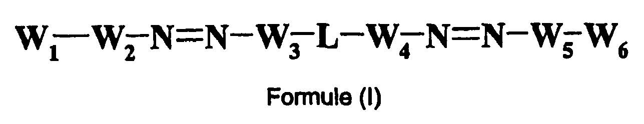 dans laquelle
- W₁ et W₆ représentent, indépendamment l'un de l'autre, un radical NR₁R₂
- W₂ et W₆ représentent, indépendamment l'un de l'autre, un groupement aromatique carboné, de formule (II) 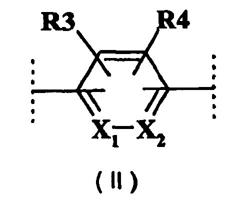
- W₃ et W₄, représentent, indépendamment l'un de l'autre, un radical hétéroaromatique représentés par les formules (III) et (IV) suivantes : 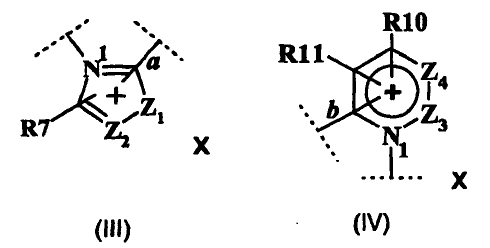 dans lesquelles
   - X₁ représente un radical CR₅,
   - X₂ représente un radical CR₆,
   - Z₁ représente un radical NR₈,
   - Z₂ représente un atome d'azote ou un radical CR₉,
   - Z₃ représente un radical CR₁₂,
   - Z₄ représente un radical CR₁₃,
   - N¹ du cycle à 5 chaînons de la formule (III) est relié au groupement L et la liaison a du même cycle à 5 chaînons est reliée au groupement azoïque de la formule (I),
   - la liaison b du cycle à 6 chaînons de la formule (IV) est reliée au groupement azoïque de la formule (I) et N¹ du cycle à 6 chaînons de la formule (IV) est relié au groupement L,
   - R₁, R₂, R₃, R₄, R₅, R₆, R₇, R₉, R₁₀, R₁₁, R₁₂ et R₁₃ représentent, ensemble ou indépendamment l'un de l'autre une chaîne hydrocarbonée en C₁-C₁₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogènes ; R₁, R₂, R₃, R₄, R₅, R₆, R₇, R₉, R₁₀, R₁₁, R₁₂ et R₁₃ peuvent représenter l'hydrogène ; R₁, R₂, R₃, R₄. R₅, R₆, R₇, R₉, R₁₀, R₁₁, R₁₂ et R₁₃ ne comportant pas de liaison peroxyde, ni de radicaux diazo ou nitroso, et L est un radical divalent, choisi parmi un radical alkyle en C₁-C₈, linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁ - C₂, carboxy ou sulfonique, de préférence un radical alkyle linéaire ou ramifié en C₁-C₈,
   - R₈ représente un radical alkyle en C₁-C₈ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique ; un radical phényle éventuellement substitué,
   - R₇ avec R₉, R₁₀ avec R₁₁ et R₁₂ avec R₁₃ peuvent former un cycle aromatique carboné, tel qu'un phényle,
   - R est un radical alkyle en C₁-C₃,
   - X est un anion organique ou minéral.

Dans le cadre de la présente invention, on entend par le terme "alkyle", sauf indication contraire un radical alkyle comprenant de 1 à 10 atomes de carbone, de préférence de 1 à 6 atomes de carbone, pouvant être linéaire ou ramifié. Le terme alcoxy signifie alkyl-O-, le terme alkyle ayant la signification précédente.

Selon l'invention, lorsqu'il est indiqué qu'un ou plusieurs des atomes de carbone de la chaîne hydrocarbonée définie pour les radicaux L, R₁, R₂, R₃, R₄, R₅, R₆, R₇, R₉, R₁₀, R₁₁, R₁₂ et R₁₃ peuvent être remplacés par un atome d'oxygène, d'azote ou de soufre ou par un groupement SO₂, et/ou que ces chaînes hydrocarbonées sont insaturées, cela signifie que l'on peut, à titre d'exemple, faire les transformations suivantes :

| | | |
|---|---|---|
| 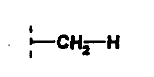 | peut devenir | 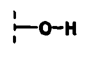 |
| 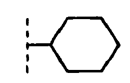 | peut devenir | 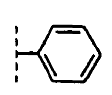 |
| 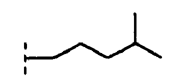 | peut devenir | 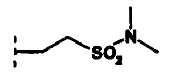 |
| 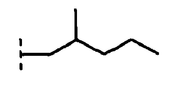 | peut devenir | 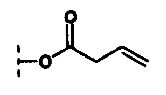 |
| 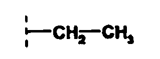 | peut devenir | 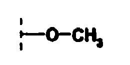 |

En particulier, on entend par "chaîne hydrocarbonée ramifiée", une chaîne pouvant former un ou plusieurs cycles carbonés comportant de 3 à 6 chaînons. On entend par chaîne hydrocarbonée insaturée, une chaîne pouvant comporter une ou plusieurs liaisons doubles et/ou une ou plusieurs liaisons triples, cette chaîne hydrocarbonée pouvant conduire à des groupements aromatiques.

X est un anion organique ou minéral par exemple choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que méthylsulfonate ; un arylsulfonate substitué ou non substitué par un radical alkyle en C₁-C₄ tel que par exemple un 4-toluylsulfonate.

R₁ et R₂ représentent, préférentiellement et indépendamment l'un de l'autre, un atome d'hydrogène ; un radical alkyle en C₁-C₆ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique ; un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome ; un radical alkylsulfonyle en C₁-C₄; un radical arylsulfonyle ; un radical choisi parmi les radicaux pyrazolyle, imidazolyle, thiazolyle, oxazolyle, thiadiazolyle, triazolyle, pyrrolyle, pyridyle, pyrimidinyle, triazinyle, pyrazinyle, pyridazinyle.

R₁ et R₂ ensemble peuvent former un radical hétérocycle à 5, 6, 7 ou 8 chaînons éventuellement substitué par un ou plusieurs radicaux hydroxy ; amino ; un groupement -NR₁₄R₁₅ dans lequel R₁₄ et R₁₅ représentent indépendamment l'un de l'autre un atome d'hydrogène, un radical alkyle en C₁-C₄ linéaire ou ramifié substitué par un ou plusieurs radicaux choisis parmi un atome d'halogène, un radical hydroxy, alcoxy en C₁-C₂, amino ou amino(di)alkyle en C₁-C₂, (poly)-hydroxyalkylamino ; un radical sulfonylamino ; un radical carboxy ; un radical carboxamido. ; un radical alkyle en C₁-C₆ éventuellement substitué par un ou plusieurs radicaux hydroxy ; amino ; un groupement -NR₁₄R₁₅.

Les carbones asymétriques desdits hétérocycles à 5,6,7 ou 8 chaînons peuvent être de configuration (R) et/ou (S).

R₁ et R₂ ensemble peuvent également formés un radical hétéroaromatique à 5 ou 6 chaînons, tels que pyrazole, imidazole, pyrrole, triazole, pyridazine, pyrimidine.

R₁ et R₂ représentent, préférentiellement et indépendamment l'un de l'autre, un atome d'hydrogène ; un radical alkyle en C₁-C₃ linéaire ou ramifié, éventuellement substitué par un ou plusieurs radicaux choisis parmi les radicaux hydroxy, alcoxy en C₁-C₂, amino, (di)alkylamino en C₁-C₂, carboxy ou sulfonique ; un radical phényle éventuellement substitué par un ou plusieurs radicaux choisis parmi les radicaux amino, (di)alkylamino en C₁-C₂ ou (poly)-hydroxyalkylamino ; R₁ et R₂ ensemble forment un hétérocycle à 5 ou 6 chaînons éventuellement substitué par un ou plusieurs radicaux choisis parmi les radicaux méthyle, hydroxy ; amino ; (dl)méthylamino, un radical sulfonylamino, un radical (poly)-hydroxyalkylamino en C₂-C₄, un radical carboxy , un radical carboxamido.

Selon un mode de réalisation particulièrement préféré, R₁ et R₂ représentent, préférentiellement et indépendamment l'un de l'autre, un atome d'hydrogène ; un radical alkyle en C₁-C₃ linéaire ou ramifié, tels que méthyle, éthyle, 2-hydroxyéthyle , 2-aminoéthyle ;1-carboxyméthyle, 2-carboxyéthyle, 2-sulfonyléthyle, 2-méthoxyéthyle ; un radical phényle éventuellement substitué par un ou plusieurs radicaux choisis parmi les radicaux amino, (di)alkylamino en C₁-C₂ , (poly)-hydroxyalkylamino ; R₁ et R₂ ensemble forment un hétérocycle à 5 ou 6 chaînons pyrrolidine ou pipéridine éventuellement substitué par un ou plusieurs radicaux méthyle , hydroxy, amino , (di)méthylamino , carboxy , carboxamido.

R₁ et R₂ représentent, préférentiellement et indépendamment l'un de l'autre, un atome d'hydrogène ; un radical méthyle, 2-hydroxyéthyle ; un radical phényle éventuellement substitué par un radical un amino, (di)méthylamino , (di)(2-hydroxyéthyl)amino; R₁ et R₂ ensemble forment un hétérocycle à 5 ou 6 chaînons tels que pyrrolidine, 3-hydroxypyrrolldine, pipéridine, 3-hydroxypipéridine.

R₃, R₄, R₅, R₆, R₁₀, R₁₁, R₁₂ et R₁₃ représentent, préférentiellement et indépendamment l'un de l'autre, un atome d'hydrogène; un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique; un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique ou un atome d'halogène tel que chlore, fluor ou brome ; un radical carboxy ; un radical sulfonylamino ; un radical sulfonique ; un radical alcoxy en C1-C2 ; un radical (poly)-hydroxyalcoxy en C₂-C₄; un radical amino ; un radical (di)alkylamino en C₁-C₂ ; un radical (poly)-hydroxyalkylamino en C₂-C_{4.}

Plus préférentiellement, R₃, R₄, R₅, R₆, R₁₀, R₁₁, R₁₂ et R₁₃ représentent un atome d'hydrogène, un radical alkyle en C₁-C₄ éventuellement substitué par un ou plusieurs radicaux choisis parmi les radicaux hydroxy, amino, (di)alkylamino en C₁-C₂ ; un radical carboxy ; un radical alcoxy en C₁-C₂ ; un radical amino ; un radical (di)alkylamino en C₁-C₂ ; un radical (poly)-hydroxyalkylamino en C₂-C₄

Selon un mode de réalisation particulièrement préféré, R₃, R₄, R₅, R_{6,} R₁₀, R₁₁, R₁₂ et R₁₃ représentent un atome d'hydrogène, un radical méthyle, phényle, 2-hydroxyméthyle, un carboxy, un radical méthoxy, éthoxy, 2-hydroxyéthyloxy, un radical amino, méthylamino, diméthylamino, 2-hydroxyéthylamino.

R₇ et R₉ représentent, indépendamment l'un de l'autre, un atome d'hydrogène ; un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle éventuellement substitué: un radical carboxy; un radical sulfonylamino.

Parmi ces substituants, R₇ et R₉ représentent préférentiellement un atome d'hydrogène, un radical phényle, un radical alkyle en C₁-C₄ éventuellement substitué par un ou plusieurs radicaux choisis parmi les radicaux hydroxy, amino, (di)alkylamino en C₁-C₂, carboxy.

Selon un mode de réalisation particulièrement préféré, R₇ et R₉ représentent préférentiellement un atome d'hydrogène, un radical méthyle, phényle, 2-hydroxyméthyle, un carboxy.

R₈ représente préférentiellement un radical alkyle en C₁-C₄ éventuellement substitué par un ou plusieurs radicaux choisis parmi les radicaux hydroxy, alcoxy en C₁-C₂, amino, (di)alkylamino en C₁-C₂ , carboxy, sulfonique. Selon un mode de réalisation particulièrement préféré, R₈ représente préférentiellement un radical méthyle, éthyle, 2-hydroxyéthyle, 1-carboxyméthyle, 2- carboxyéthyle, 2-sulfonyléthyle.

Selon un mode de réalisation particulier, W1 et W6 sont NR₁R₂ et R₁ et R₂ sont de préférence choisis parmi l'hydrogène, un radical alkyle pouvant être substitué par un hydroxy ou un amino.

Selon un autre mode de réalisation, W1 et W6 sont OR et R est de préférence un radical méthyle.

Dans la formule (I), W2 et W5 sont des radicaux aromatiques carbonés. De préférence, W2 et W5 représentent un radical phényle éventuellement substitué par un radical alkyle, alcoxy, amino, amino mono ou di substitué. A titre d'exemple, W2 et W5 sont des radicaux phényle substitués par un radical méthoxy, un radical méthyl, un radical amino, un radical méthylamino, un radical diméthylamino.

W3 et W4 sont de préférence choisis parmi les radicaux imidazolinium, triazollum, pyridinium.

Selon un mode de réalisation particulièrement préféré, W2 et W5 sont des radicaux phénylènes, W3 et W4 sont des radicaux imidazolinium.

Dans la formule (I), L est de préférence un radical alkylène en C₁-C₈, linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁ -C₂ , carboxy ou sulfonique; de préférence un radical alkylène linéaire ou ramifié en C₁-C₈. Selon un mode de réalisation préféré, L est un radical alkylène en C₁-C₈, linéaire ou ramifié, éventuellement substitué par un ou plusieurs radicaux choisis parmi les radicaux hydroxy, alcoxy en C₁-C₂, amino, (di)alkylamino en C₁-C₂.

La concentration en colorant dicationique diazoique de formule (I) peut varier entre 0,001 et 5% en poids environ par rapport au poids total de la composition tinctoriale, et de préférence entre environ 0,05 et 2%.

La composition de l'invention peut de plus comprendre un agent oxydant. Cet agent oxydant peut être n'importe quel agent oxydant utilisé de façon classique pour la décoloration des fibres kératiniques. L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition selon l'invention peut de plus comprendre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(P-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthy;oxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05163124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3.7-diamlne ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazoio-[1,5-a]-pyrimidine-3,5-diamine ; le 3-amino pyrazolo-[1,5-a]-pyrimidin-7-ol ; le 3-amino pyrazolo-[1,5-a]-pyrimidin-5-oi ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4.5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4.5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir de plus un ou plusieurs coupleurs conventionnellement utilisés pour la teinture de fibres kératiniques. Parmi ces coupleurs, on peut notamment citer les métaphénylènediamines, les métaaminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général présents en quantité comprise entre 0,001 et 10 % en poids environ du poids total de la composition tinctoriale et plus préférentiellement de 0,005 à 6 %. La ou les bases d'oxydation sont présentes en quantité de préférence comprise entre 0,001 à 10 % en poids environ du poids total de la composition tinctoriale, et plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir des colorants directs différents de ceux de formule (I), ces colorants pouvant notamment être choisis parmi les colorants nitrés de la série benzénique, les colorants directs cationiques, les colorants directs azoïques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 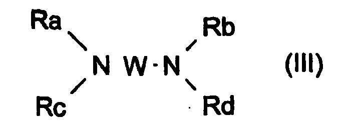 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe qui comprend l'application d'une composition tinctoriale contenant un colorant de formule (I) telle que définie précédemment sur les fibres kératiniques. Après un temps de pause, les fibres kératiniques sont rincées laissant apparaître des fibres colorées.

L'application sur les fibres de la composition tinctoriale contenant le colorant cationique azoïque de formule (I) peut être mise en oeuvre en présence d'agent oxydant ce qui provoque la décoloration de la fibre. Cet agent oxydant peut être ajouté à la composition contenant le colorant cationique azoïque au moment de l'emploi ou directement sur la fibre kératinique. Selon un mode de réalisation particulier, la composition contenant le colorant cationique azoïque de formule (I) est exempte de base d'oxydation et de coupleur.

L'invention a aussi pour objet un procédé de teinture d'oxydation permanente qui comprend l'application sur les fibres d'une composition tinctoriale qui comprend un colorant de formule (I), au moins une base d'oxydation et optionnellement au moins un coupleur, en présence d'un agent oxydant.

La base d'oxydation, le coupleur et l'agent oxydant sont tels que définis précédemment.

Dans le cadre de la teinture d'oxydation permanente, il est aussi possible d'utiliser comme agent oxydant des enzymes parmi lesquelles on peut citer les peroxydases, les oxydo-réductases à 2 électrons telles que les uricases et les oxygénases à 4 électrons comme les laccases.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée sur les fibres simultanément ou séquentiellement à la composition tinctoriale.

Dans le cas de la teinture d'oxydation permanente ou de la teinture directe, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Enfin l'invention a également pour objet les colorants diazoïques dicationiques de formule (I) telle que définie précédemment. Ces composés peuvent être obtenus à partir des procédés de préparation décrits par exemple dans les documents EP 810824, GB 9619573, RO 106572, J.Chem. Res.., Synop. (1998), (10), 648-649, DE 19721619, US 5852179, Synth. Commun 1999, 29(13), 2271-2276.

Les exemples qui suivent servent à illustrer l'invention sans toutefois présenter un caractère limitatif.

### EXEMPLES

### EXEMPLES DE SYNTHESE

### Exemple n°1:préparation d'un composé de formule :

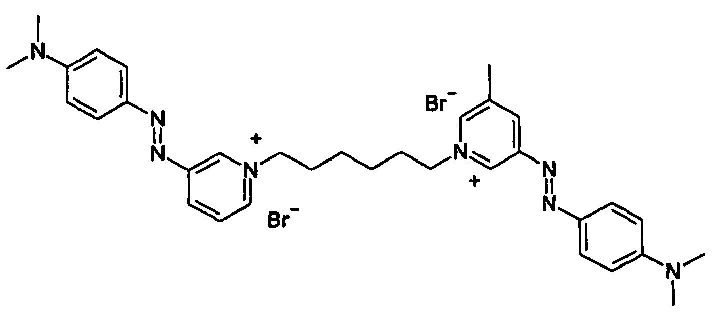

Dans un ballon tout équipé, on charge 1,13g de dimethyl-[4-(pyridin-3-ylazo)-phenyl1]-amine (0.01mole), 5ml de DMF sec et 0,61g (0,0025mole) de 1,6-dibromohexane. Le mélange est porté 7 heures à 105°C sous agitation. On filtre à chaud le précipité. On obtient 0,9g de poudre rouge bordeaux présentant les caractéristiques suivantes en absorption UV :

| | |
|---|---|
| UV (acétonitrile-eau 50/50) | λₘₐₓ = 496 nm |
| | εₘₐₓ = 67600 |

### Analyses :

| | |
|---|---|
| Masse ESI+ : | m/z = 617[M²⁺ + Br⁻] |
| | m/z = 268[M²⁺/2] |

RMN 1H : (400MHz-DMSO) ppm :
1.40(s-4H) ;2.01(s-4H) CH₂ hexyl
3.15(s-12H)-NCH₃; 4.73(t-4H)-NCH₂ ;
6.91(d-4H) ; 7.88(d-4H) phenyl
8.33(dd-2H) ;8.76(d-2H) ; 9.11(d-2H) ; 9.54(s-2H) pyridine

On obtient ainsi un colorant donnant une teinture rouge-orange intense

Le dimethyl-[4-(pyridin-3-ylazo)-phenyl1]-amine peut être préparé par copulation du sel de diazonium de l'amino-3-pyridine sur la dimethylaniline suivant selon les methodes classiques de préparations d'azoiques ou suivant l'enseignement du brevet DE2509560.

### Exemple n°2 : préparation d'un composé de formule :

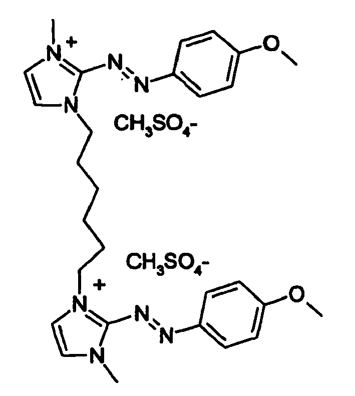

### 1^{ère} étape : 1,6-bis[N-imidazol-2-yl)-4(4-methoxy-phenyl)diazene] :

Dans un ballon tout équipé, on charge 2,02g (0,01mole) de (1H-imidazol-2-yl)-(4-methoxy-phenyl)-diazene, 50ml de toluène et 1,21g (0,005mole) de 1,6-dibromohexane,1,92g (0,048mole) de soude, (0.0003mole) d'iodure de tetrabutylammonium et 2ml d'eau Le mélangé est porté 20 heures au reflux sous agitation. On refroidit, filtre et sèche le précipité On obtient 0,9g de poudre jaune orangé que l'on purifie par chromatographie sur silice (éluant :dichloromethane 95 /méthanol 5)

### Analyse :

Masse ESI+ : m/z = 487[M⁺⁻]
RMN 1H : (400MHz-DMSO) ppm :
   1.27(s-4H ) ;1.74(s-4H ) CH₂ hexyl
   3.85(s-6H)-OCH₃; 4.33(t-4H)-NCH₂ ;
   7.16(d-4H) ; 7.82(d-4H) phenyl
   7.51(s-2H) imidazole

La préparation de ce type de diamines est décrite dans la demande de brevet WO 99/37276 et bien dans les documents suivants : J.Elguero et All, Journal of Hétérocyclic Chemistry 2-5, 771-782 (1988) Ying-hung So, Macromolécules 25, 516-520 (1992) et R.G.Xie and all, Chinese Chemical Letters 7, 321-324 (1996)

### • 2^{ème} étape :quaternisation :

Dans un ballon tout équipé, on charge 0,6g (0,0012mole) du produit obtenu précedement, 4ml de dichloroéthane et 0,47ml (0,0048 mole) de sulfate de diméthyle. Le mélange est porté 2 heures au reflux sous agitation. On refroidit, filtre et sèche le précipité On obtient 0,6g de poudre jaune orangé présentant les caractéristiques suivantes en absorption UV :

| | |
|---|---|
| UV (éthanol) | λₘₐₓ = 406 nm épaulement à 450nm |
| | εₘₐₓ = 53400 |

RMN 1H : (400MHz-DMSO) ppm :
1.37(s-4H);1.79(s-4H) CH₂ hexyle
3.94(s-6H)-OCH₃; 4.45(t-4H)-NCH₂ ;4.04(s-6H)-NCH3
7.24(d-4H) ; 7.07(d-4H) phenyl
7.96(d-4H) imidazole

On obtient ainsi un colorant donnant une teinture Jaune Intense

### Exemple n°3 : préparation d'un composé de formule :

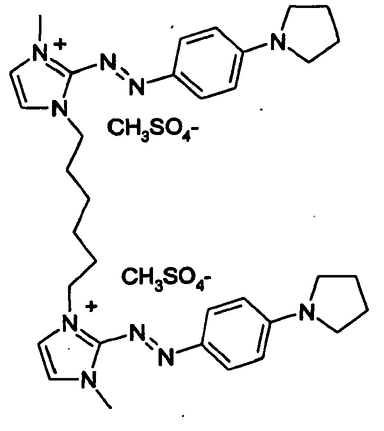

Dans un ballon tout équipé, on charge 0,3g du produit obtenu précedement (0,0004mole), 2ml de DMF sec et 0,57g (0,008mole) de pyrrolidine Le mélange est porté 1,5 heure à 100°C sous agitation. On verse le mélange réactionnel dans 50ml d'acétate d'éthyle. Le précipité est esssoré et séché .On obtient 0,4g de poudre rouge bordeaux présentant les caractéristiques suivantes en absorption UV :

| | |
|---|---|
| UV (éthanol) | λₘₐₓ = 534 nm |
| | εₘₐₓ = 86000 |

RMN 1H : (400MHz-DMSO) ppm :
1.35(s-4H ) ;1.77(s-4H ) CH₂ hexyle
2.02(m-8H ) ;3.44(massif HDO+8H) pyrrolidine
4.32(t-4H)-NCH₂ ;3.92(s-6H)-NCH3
6.77(d-4H) ; 7.84(d-4H) phenyl
7.69(d-2H) ; 7.72(d-4H)imidazole

On obtient ainsi un colorant donnant une teinture rouge intense.

### EXEMPLES DE TEINTURE

On a préparé les compositions tinctoriales suivantes

| Exemple | 4 | 5 |
|---|---|---|
| Colorant azoïque de l'exemple 1 | 5x10⁻⁴ mole | - |
| Colorant de l'exemple 3 | - | 5x10⁻⁴ mole |
| Polyéthylène glycol 8 OE | 12 g | 12 g |
| Alcool benzylique | 10 g | 10 g |
| Tampon Borate q.s.p | 100 g | 100 g |
| pH | 8,9 | 9,1 |

Au moment de l'emploi, chaque composition est mélangée avec un poids égal d'eau oxygénée à 20 volumes (6% en poids).

Chaque mélange obtenu est appliqué sur des mèches de cheveux gris à 90 % de blancs, permanentés (BP) ou naturel (BN) (1 g de mèche pour 10 g de solution). Après 20 min de pose, les mèches sont rinçées, lavées avec un shampooing standard, rinçées à nouveau puis séchées.

Chaque mèche est évaluée avant et après la teinture dans le système L*a*b*, au moyen d'un spectrophotomètre CM 2002 MINOLTA ®, (Illuminant D65).

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu.

Selon ce système, plus la valeur de L est élevée, plus la couleur est claire et peu intense. Inversement, plus la valeur de L est faible, plus la couleur est foncée ou très intense.

Les résultats de teinture suivants ont été obtenus.

| | **Cheveux naturels** | | | **Cheveux permanentés** | | |
|---|---|---|---|---|---|---|
| | **L*** | **a*** | **b*** | **L*** | **a*** | **b*** |
| **Exemple 4** | 23,6 | 15,8 | 11,2 | 22,5 | 15,7 | 10,1 |
| **Exemple 5** | 21,8 | 16,7 | 7,0 | 20,1 | 13,9 | 4,5 |

## Revendications

1. Composition pour la teinture des fibres kératiniques humaines, comprenant au moins un colorant diazoïque dicationique de formule (I) suivante : comprenant au moins un 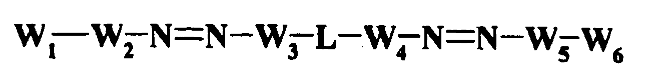 dans laquelle
- W₁ et W₆ représentent, indépendamment l'un de l'autre, un radical NR₁R₂ ou OR,
- W₂ et W₅ représentent, indépendamment l'un de l'autre, un groupement aromatique carboné, de formule (II) 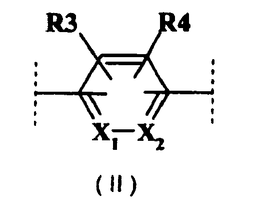
- W₃ et W_{4,} représentent, indépendamment l'un de l'autre, un radical hétéroaromatique représentés par les formules (III) et (IV) suivantes : 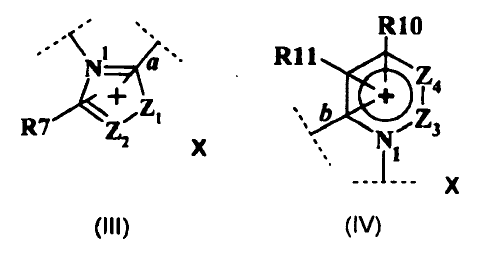 dans lesquelles
- X₁ représente un radical CR_{5,}
- X₂ représente un radical CR_{6,}
- Z₁ représente un radical NR_{8,}
- Z₂ représente un atome d'azote ou un radical CR_{9,}
- Z₃ représente un radical CR_{12,}
- Z₄ représente un radical CR_{13,}
- N¹ du cycle à 5 chaînons de la formule (III) est relié au groupement L et la liaison a du même cycle à 5 chaînons est reliée au groupement azoïque de la formule (I),
- la liaison b du cycle à 6 chaînons de la formule (IV) est reliée au groupement azoïque de la formule (I) et N¹ du cycle à 6 chaînons de la formule (IV) est relié au groupement L,
- R₁, R₂, R₃, R₄, R₅, R₆, R₇, R₉, R₁₀, R₁₁, R₁₂ et R₁₃ représentent, ensemble ou indépendamment l'un de l'autre une chaîne hydrocarbonée en C₁-C₁₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogènes ; R₁, R₂, R₃, R₄, R₅, R₆, R₇, R₉, R₁₀, R₁₁, R₁₂ et R₁₃ peuvent représenter l'hydrogène : R₁, R₂, R₃, R₄, R₅, R₆, R₇, R₉, R₁₀, R₁₁, R₁₂ et R₁₃ ne comportant pas de liaison peroxyde, ni de radicaux diazo ou nitroso, et L est un radical divalent, choisi parmi un radical alkyle en C₁-C₈, linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁ - C₂ , carboxy ou sulfonique, de préférence un radical alkyle linéaire ou ramifié en C₁-C₈,
- R₈ représente un radical alkyle en C₁-C₈ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique ; un radical phényle éventuellement substitué,
- R₇ avec R₉, R₁₀ avec R₁₁ et R₁₂ avec R₁₃ peuvent former un cycle aromatique carboné,
- R est un radical alkyle en C₁- C_{3,}
- X est un anion organique ou minéral.

2. Composition selon la revendication 1 dans laquelle R₁ et R₂ représentent un atome d'hydrogène ; un radical alkyle en C₁-C₆ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique ; un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique ou un atome d'halogène ; un radical alkylsulfonyle en C₁-C₄; un radical arylsulfonyle ; un radical choisi parmi les radicaux pyrazolyle, imidazolyle, thiazolyle, oxazolyle, thiadiazolyle, triazolyle, pyrrolyle, pyridyle, pyrimidinyle, triazinyle, pyrazinyle, pyridazinyle ; R₁ et R₂ ensemble forment un hétérocycle à 5, 6, 7 ou 8 chaînons éventuellement substitué par un ou plusieurs radicaux hydroxy ; amino ; -NR₁₄R₁₅ dans lequel R₁₄ et R₁₅ représentent indépendamment l'un de l'autre un atome d'hydrogène, un radical alkyle en C₁-C₄ linéaire ou ramifié substitué par un ou plusieurs radicaux choisis parmi un atome d'halogène, un radical hydroxy, alcoxy en C₁-C₂, amino ou amino(di)alkyle en C₁-C₂ , (poly)-hydroxyalkylamino ; un radical sulfonylamino ; un radical carboxy ; un radical carboxamido ; un radical alkyle en C₁-C₆ éventuellement substitué par un ou plusieurs radicaux hydroxy ; amino ; un groupement -NR₁₄R₁₅ ; R₁ et R₂ ensemble forment un radical hétéroaromatique à 5 ou 6 chaînons .

3. Composition selon la revendication 2 dans laquelle R₁ et R₂ représentent un atome d'hydrogène; un radical alkyle en C₁-C₃ linéaire ou ramifié, éventuellement substitué par un ou plusieurs radicaux choisis parmi les radicaux hydroxy, alcoxy en C₁-C₂, amino, (di)alkylamino en C₁-C₂ , carboxy ou sulfonique ; un radical phényle éventuellement substitué par un ou plusieurs radicaux choisis parmi les radicaux amino, (di)alkylamino en C₁-C₂ ou (poly)-hydroxyalkylamino ; R₁ et R₂ ensemble forment un hétérocycle à 5 ou 6 chaînons éventuellement substitué par un ou plusieurs radicaux choisis parmi les radicaux méthyle, hydroxy; amino ; (di)méthylamino ; un radical sulfonylamino, un radical (poly)-hydroxyalkylamino en C₂-C₄ , un radical carboxy , un radical carboxamido.

4. Composition selon la revendication 3 dans laquelle R₁ et R₂ représentent un atome d'hydrogène ; méthyle, éthyle, 2-hydroxyéthyle , 2-aminoéthyle ;1-carboxyméthyle, 2-carboxyéthyle, 2-sulfonyléthyle, 2-méthoxyéthyle ; un radical phényle éventuellement substitué par un ou plusieurs radicaux choisis parmi les radicaux amino, (di)alkylamino en C₁-C₂ , (poly)-hydroxyalkylamino ; R₁ et R₂ ensemble forment un hétérocycle à 5 ou 6 chaînons pyrrolidine ou pipéridine éventuellement substitué par un ou plusieurs radicaux méthyle, hydroxy, amino, (di)méthylamino , carboxy , carboxamido.

5. Composition selon la revendication 4 dans laquelle R₁ et R₂ représentent un atome d'hydrogène ; un radical méthyle, un radical 2-hydroxyéthyle ; un radical phényle éventuellement substitué par un radical un amino, (di)méthylamino , (di)(2-hydroxyéthyl)amino ; R₁ et R₂ ensemble forment une pyrrolidine, 3-hydroxypyrrolidine, pipéridine, 3-hydroxypipéridine ; R₁ et R₂ ensemble forment une pyrazole, imidazole, pyrrole, triazole, pyridazine, pyrimidine.

6. Composition selon l'une quelconque des revendications 1 à 5 dans laquelle R₃, R₄, R₅, R_{6,} R₁₀, R₁₁, R₁₂ et R₁₃ représentent un atome d'hydrogène ; un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique; un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique ou un atome d'halogène ; un radical carboxy ; un radical sulfonylamino ; un radical sulfonique ; un radical alcoxy en C1-C2 ; un radical (poly)-hydroxyalcoxy en C₂-C₄ ; un radical amino ; un radical (di)alkylamino en C₁-C₂ ; un radical (poly)-hydroxyalkylamino en C₂-C₄.

7. Composition selon la revendication 6 dans laquelle R₃, R₄, R₅, R_{6.} R₁₀, R₁₁, R₁₂ et R₁₃ représentent un atome d'hydrogène, un radical alkyle en C₁-C₄ éventuellement substitué par un ou plusieurs radicaux choisis parmi les radicaux hydroxy, amino, (di)alkylamino en C₁-C₂ ; un radical carboxy ; un radical alcoxy en C₁-C₂ ; un radical amino ; un radical (di)alkylamino en C₁-C₂ ; un radical (poly)-hydroxyalkylamino en C₂-C₄.

8. Composition selon la revendication 7 dans laquelle R₃, R₄, R₅, R_{6,} R₁₀, R₁₁, R₁₂ et R₁₃ représentent un atome d'hydrogène, un radical méthyle, phényle, 2-hydroxyméthyle, un carboxy, un radical méthoxy, éthoxy, 2-hydroxyéthyloxy, un radical amino, méthylamino, diméthylamino, 2-hydroxyéthylamino.

9. Composition selon l'une quelconque des revendications 1 à 8 dans laquelle R₇ et R₉ représentent un atome d'hydrogène ; un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle éventuellement substitué ; un radical carboxy ; un radical sulfonylamino.

10. Composition selon la revendication 9 dans laquelle R₇ et R₉ représentent un atome d'hydrogène, un radical phényle, un radical alkyle en C₁-C₄ éventuellement substitué par un ou plusieurs radicaux choisis parmi les radicaux hydroxy, amino, (di)alkylamino en C₁-C₂ , carboxy.

11. Composition selon la revendication 10 dans laquelle R₇ et R₉ représentent un atome d'hydrogène, un radical méthyle, phényle, 2-hydroxyméthyle, carboxy.

12. Composition selon l'une quelconque des revendications 1 à 11 dans laquelle R₈ représente un radical alkyle en C₁-C₄ éventuellement substitué par un ou plusieurs radicaux choisis parmi les radicaux hydroxy, alcoxy en C₁-C₂, amino, (di)alkylamino en C₁-C₂ , carboxy, sulfonique.

13. Composition selon la revendication 12 dans laquelle R₈ représente un radical méthyle, éthyle, 2-hydroxyéthyle, 1-carboxyméthyle, 2- carboxyéthyle, 2-sulfonyléthyle.

14. Composition selon l'une quelconque des revendications 1 à 13 dans laquelle W1 et W6 sont NR1R2 et R1, et R2 sont choisis parmi l'hydrogène, un radical alkyle pouvant être substitué par un hydroxy ou un amino.

15. Composition selon l'une quelconque des revendications 1 à 13 dans laquelle W1 et W6 sont OR et R est un radical méthyle.

16. Composition selon la revendication 1 dans laquelle W2 et W5 représentent un radical phényle éventuellement substitué par un radical alkyle, alcoxy, amino, amino mono ou di substitué, méthoxy, un radical méthyl, un radical amino, un radical méthylamino, un radical diméthylamino.

17. Composition selon l'une quelconque des revendications précédentes dans laquelle W3 et W4 sont choisis parmi les radicaux imidazolinium, triazolium, pyridinium.

18. Composition selon l'une quelconque des revendications précédentes dans laquelle le colorant de formule (I) est tel que
• W2 et W5 sont des radicaux phénylènes,
• W3 et W4 sont des radicaux imidazolinium.

19. Composition selon l'une quelconque des revendications 1 à 18 dans laquelle L est un radical alkylène en C₁-C₈, linéaire ou ramifié, éventuellement substitué par un ou plusieurs radicaux choisis parmi les radicaux hydroxy, alcoxy en C₁-C₂, amino, (di)alkylamino en C₁ -C₂.

20. Composition selon l'une quelconque des revendications 1 à 19 dans laquelle le colorant de formule (I) est choisi parmi

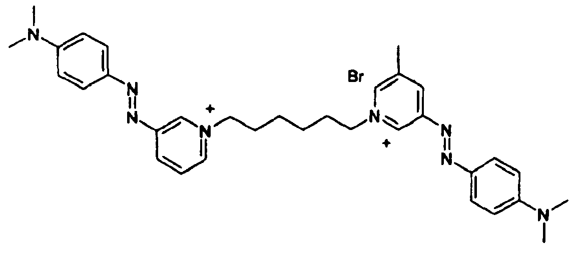

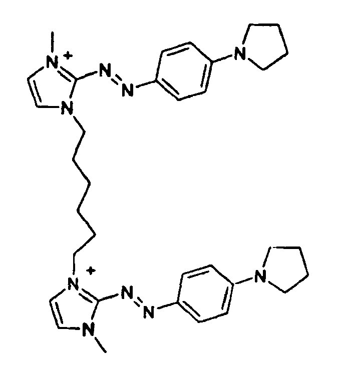

21. Composition selon l'une quelconque des revendications 1 à 19 dans laquelle le colorant est

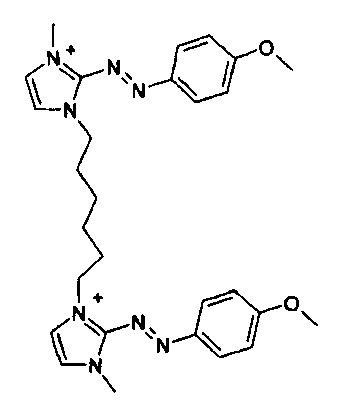

22. Composition selon l'une quelconque des revendications 1 à 21 comprenant de plus une base d'oxydation.

23. Composition selon la revendication 22 dans laquelle la base d'oxydation est choisie parmi les paraphénylènediamines, les bis-phénylalkylènediamines, les para-aminophénols, les ortho-aminophénols, les bases hétérocycliques, et leurs sels d'addition avec un acide.

24. Composition selon l'une quelconque des revendications 22 ou 23 dans laquelle la ou les bases d'oxydation sont présentes en quantité comprise entre 0,001 et 10 %, de préférence entre 0,005 et 6 %.

25. Composition selon l'une quelconque des revendications 1 à 24 comprenant au moins un coupleur.

26. Composition selon la revendication 25 dans laquelle le coupleur est choisi parmi métaphénylènediamines, les méta-aminophénols, les métadiphénols, les coupleurs naphtaléniques et les coupleurs hétérocycliques et leur sel d'addition avec un acide.

27. Composition selon l'une quelconque des revendications 1 à 26 comprenant de plus un agent oxydant, de préférence le peroxyde d'hydrogène.

28. Procédé de teinture d'oxydation des fibres kératiniques et en particulier des fibres kératiniques humaines telles que les cheveux, **caractérisé en ce qu'**on applique sur les fibres au moins une composition tinctoriale telle que définie à l'une quelconque des revendications 1 à 26.

29. Procédé selon la revendication 28 dans lequel la composition tinctoriale contient un agent oxydant.

30. Procédé selon la revendication 29 dans lequel l'agent oxydant est mélangé au moment de l'emploi à la composition tinctoriale.

31. Procédé selon l'une quelconque des revendications 29 ou 30 dans lequel l'agent oxydant est appliqué sur les fibres sous forme de composition oxydante simultanément ou séquentiellement à la composition tinctoriale.

32. Procédé de teinture d'oxydation des fibres kératiniques humaines, **caractérisé en ce qu'**on applique sur les fibres au moins une composition tinctoriale telle que définie selon l'une quelconque des revendications 1 à 21, comprenant de plus au moins une base d'oxydation et optionnellement au moins un coupleur en présence d'un agent oxydant.

33. Procédé selon la revendication 32 dans lequel l'agent oxydant est mélangé au moment de l'emploi à la composition tinctoriale.

34. Procédé selon la revendication 32 dans lequel l'agent oxydant est appliqué sur les fibres sous forme de composition oxydante simultanément ou séquentiellement à la composition tinctoriale.

35. Dispositif à plusieurs compartiments ou "kit" de teinture à plusieurs compartiments, dans lequel un premier compartiment contient une composition telle que définie à l'une quelconque des revendications 1 à 26 et un deuxième compartiment contient une composition oxydante.

## Claims

1. Composition for dyeing human keratinous fibers, comprising at least one dicationic diazo dye of the following formula (I): 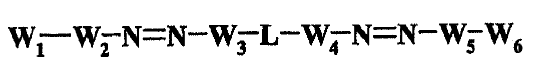 in which
- W₁ and W₆ represent, independently of one another, a radical NR₁R₂ or OR,
- W₂ and W₅ represent, independently of one another, a carbonaceous aromatic group of formula (II) 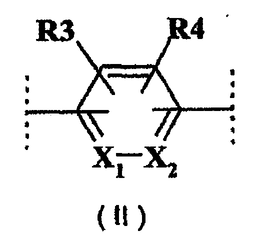
- W₃ and W₄ represent, independently of one another, a heteroaromatic radical represented by the following formulae (III) and (IV): 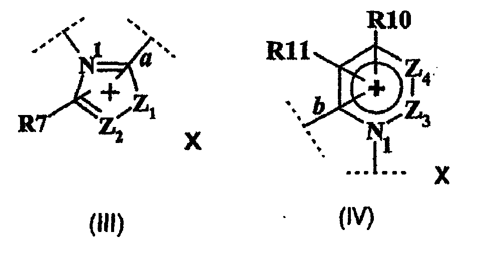 in which
- X₁ represents a radical CR₅,
- X₂ represents a radical CR₆,
- Z₁ represents a radical NR₈,
- Z₂ represents a nitrogen atom or a radical CR₉,
- Z₃ represents a radical CR₁₂,
- Z₄ represents a radical CR₁₃,
- N¹ of the 5-membered ring of formula (III) is connected to the group L and the bond ***a*** of the same 5-membered ring is connected to the azo group of formula (I),
- the bond ***b*** of the 6-membered ring of formula (IV) is connected to the azo group of formula (I) and N¹ of the 6-membered ring of formula (IV) is connected to the group L,
- R₁, R₂, R₃, R₄, R₅, R₆, R₇, R₉, R₁₀, R₁₁, R₁₂ and R₁₃ represent, together or independently of one another, a linear or branched C₁-C₁₆ hydrocarbonaceous chain, which can form one or more 3- to 6-membered carbonaceous rings, and which can be saturated or unsaturated, of which one or more carbon atoms of the carbonaceous chain can be replaced by an oxygen, nitrogen or sulfur atom or by an SO₂ group, and the carbon atoms of which can be, independently of one another, substituted by one or more halogen atoms; R₁, R₂, R₃, R₄, R₅, R₆, R₇, R₉, R₁₀, R₁₁, R₁₂ and R₁₃ can represent hydrogen; R₁, R₂, R₃, R₄, R₅, R₆, R₇, R₉, R₁₀, R₁₁, R₁₂ and R₁₃ not comprising a peroxide bond, or diazo or nitroso radicals, and L is a divalent radical chosen from a linear or branched C₁-C₈ alkyl radical optionally substituted by one or more radicals chosen from the hydroxyl, C₁-C₂ alkoxy, C₂-C₄ (poly)hydroxyalkoxy, amino, C₁-C₂ (di)alkylamino, carboxyl or sulfonic radicals, preferably a linear or branched C₁-C₈ alkyl radical,
- R₈ represents a linear or branched C₁-C₈ alkyl radical optionally substituted by one or more radicals chosen from hydroxyl, C₁-C₂ alkoxy, C₂-C₄ (poly)hydroxyalkoxy, amino, C₁-C₂ (di)-alkylamino, carboxyl or sulfonic radicals; an optionally substituted phenyl radical,
- R₇ with R₉, R₁₀ with R₁₁ and R₁₂ with R₁₃ can form a carbonaceous aromatic ring,
- R is a C₁-C₃ alkyl radical,
- X is an organic or inorganic anion.

2. Composition according to Claim 1, in which R₁ and R₂ represent a hydrogen atom; a linear or branched C₁-C₆ alkyl radical, optionally substituted by one or more radicals chosen from hydroxyl, C₁-C₂ alkoxy, C₂-C₄ (poly)hydroxyalkoxy, amino, C₁-C₂ (di)alkylamino, carboxyl or sulfonic radicals; a phenyl radical optionally substituted by one or more radicals chosen from hydroxyl, C₁-C₂ alkoxy, C₂-C₄ (poly)hydroxyalkoxy, amino, C₁-C₂ (di)alkylamino, carboxyl or sulfonic radicals or a halogen atom; a C₁-C₄ alkylsulfonyl radical; an arylsulfonyl radical; a radical chosen from pyrazolyl, imidazolyl, thiazolyl, oxazolyl, thiadiazolyl, triazolyl, pyrrolyl, pyridyl, pyrimidinyl, triazinyl, pyrazinyl and pyridazinyl radicals; R₁ and R₂ together form a 5-, 6-, 7- or 8-membered heterocycle optionally substituted by one or more hydroxyl or amino radicals; -NR₁₄R₁₅ in which R₁₄ and R₁₅ represent, independently of one another, a hydrogen atom, a linear or branched C₁-C₄ alkyl radical substituted by one or more radicals chosen from a halogen atom, a hydroxyl, C₁-C₂ alkoxy, amino or C₁-C₂ amino(di)alkyl, or (poly)hydroxyalkylamino radical; a sulfonylamino radical; a carboxyl radical; a carboxamido radical; a C₁-C₆ alkyl radical optionally substituted by one or more hydroxyl or amino radicals; a group -NR₁₄R₁₅; R₁ and R₂ together form a 5- or 6-membered heteroaromatic radical.

3. Composition according to Claim 2, in which R₁ and R₂ represent a hydrogen atom; a linear or branched C₁-C₃ alkyl radical optionally substituted by one or more radicals chosen from the hydroxyl, C₁-C₂ alkoxy, amino, C₁-C₂ (di)alkylamino, carboxyl or sulfonic radicals; a phenyl radical optionally substituted by one or more radicals chosen from amino, C₁-C₂ (di)alkylamino or (poly)hydroxyalkylamino radicals; R₁ and R₂ together form a 5- or 6-membered heterocycle optionally substituted by one or more radicals chosen from methyl, hydroxyl, amino or (di)methylamino radicals, a sulfonylamino radical, a C₂-C₄ (poly)hydroxyalkylamino radical, a carboxyl radical or a carboxamido radical.

4. Composition according to Claim 3, in which R₁ and R₂ represent a hydrogen atom; methyl, ethyl, 2-hydroxyethyl, 2-aminoethyl; 1-carboxymethyl, 2-carboxyethyl, 2-sulfonylethyl, 2-methoxyethyl; a phenyl radical optionally substituted by one or more radicals chosen from amino, C₁-C₂ (di)alkylamino or (poly)hydroxyalkylamino radicals; R₁ and R₂ together form a 5- or 6-membered heterocycle pyrrolidine or piperidine optionally substituted by one or more methyl, hydroxyl, amino, (di)methylamino, carboxyl or carboxamido radicals.

5. Composition according to Claim 4, in which R₁ and R₂ represent a hydrogen atom; a methyl or 2-hydroxyethyl radical; a phenyl radical optionally substituted by an amino, (di)methylamino or (di)(2-hydroxyethyl)amino radical; R₁ and R₂ together form a pyrrolidine, 3-hydroxypyrrolidine, piperidine or 3-hydroxypiperidine; R₁ and R₂ together form a pyrazole, imidazole, pyrrole, triazole, pyridazine or pyrimidine.

6. Composition according to any one of Claims 1 to 5, in which R₃, R₄, R₅, R₆, R₁₀, R₁₁, R₁₂ and R₁₃ represent a hydrogen atom; a linear or branched C₁-C₄ alkyl radical optionally substituted by one or more radicals chosen from the hydroxyl, C₁-C₂ alkoxy, C₂-C₄ (poly)hydroxyalkoxy, amino, C₁-C₂ (di)alkylamino, carboxyl or sulfonic radicals; a phenyl radical optionally substituted by one or more radicals chosen from hydroxyl, C₁-C₂ alkoxy, C₂-C₄ (poly)hydroxyalkoxy, amino, C₁-C₂ (di)alkylamino, carboxyl or sulfonic radicals or a halogen atom; a carboxyl radical; a sulfonylamino radical; a sulfonic radical; a C₁-C₂ alkoxy radical; a C₂-C₄ (poly)hydroxyalkoxy radical; an amino radical; a C₁-C₂ (di)alkylamino radical; a C₂-C₄ (poly)hydroxyalkylamino radical.

7. Composition according to Claim 6, in which R₃, R₄, R₅, R₆, R₁₀, R₁₁, R₁₂ and R₁₃ represent a hydrogen atom, a C₁-C₄ alkyl radical optionally substituted by one or more radicals chosen from the hydroxyl, amino or C₁-C₂ (di)alkylamino radicals; a carboxyl radical; a C₁-C₂ alkoxy radical; an amino radical; a C₁-C₂ (di)alkylamino radical; a C₂-C₄ (poly)hydroxyalkylamino radical.

8. Composition according to Claim 7, in which R₃, R₄, R₅, R₆, R₁₀, R₁₁, R₁₂ and R₁₃ represent a hydrogen atom, a methyl, phenyl or 2-hydroxymethyl radical, a carboxyl, a methoxy, ethoxy or 2-hydroxyethyloxy radical, an amino, methylamino, dimethylamino or 2-hydroxyethylamino radical.

9. Composition according to any one of Claims 1 to 8, in which R₇ and R₉ represent a hydrogen atom; a linear or branched C₁-C₄ alkyl radical, optionally substituted by one or more radicals chosen from the hydroxyl, C₁-C₂ alkoxy, C₂-C₄ (poly)hydroxyalkoxy, amino, C₁-C₂ (di)alkylamino, carboxyl or sulfonic radicals; an optionally substituted phenyl radical; a carboxyl radical; a sulfonylamino radical.

10. Composition according to Claim 9, in which R₇ and R₉ represent a hydrogen atom, a phenyl radical, a C₁-C₄ alkyl radical optionally substituted by one or more radicals chosen from the hydroxyl, amino, C₁-C₂ (di)alkylamino and carboxyl radicals.

11. Composition according to Claim 10, in which R₇ and R₉ represent a hydrogen atom, a methyl, phenyl, 2-hydroxymethyl or carboxyl radical.

12. Composition according to any one of Claims 1 to 11, in which R₈ represents a C₁-C₄ alkyl radical optionally substituted by one or more radicals chosen from hydroxyl, C₁-C₂ alkoxy, amino, C₁-C₂ (di)alkylamino, carboxyl and sulfonic radicals.

13. Composition according to Claim 12, in which R₈ represents a methyl, ethyl, 2-hydroxyethyl, 1-carboxymethyl, 2-carboxyethyl or 2-sulfonylethyl radical.

14. Composition according to any one of Claims 1 to 13, in which W1 and W6 are NR1R2 and R1 and R2 are chosen from hydrogen, an alkyl radical which can be substituted by a hydroxyl or an amino.

15. Composition according to any one of Claims 1 to 13, in which W1 and W6 are OR and R is a methyl radical.

16. Composition according to Claim 1, in which W2 and W5 represent a phenyl radical optionally substituted by an alkyl, alkoxy, amino, mono- or disubstituted amino, or methoxy radical, a methyl radical, an amino radical, a methylamino radical or a dimethylamino radical.

17. Composition according to any one of the preceding claims, in which W3 and W4 are chosen from imidazolinium, triazolium and pyridinium radicals.

18. Composition according to any one of the preceding claims, in which the dye of formula (I) is such that
• W2 and W5 are phenylene radicals,
• W3 and W4 are imidazolinium radicals.

19. Composition according to any one of Claims 1 to 18, in which L is a linear or branched C₁-C₈ alkylene radical optionally substituted by one or more radicals chosen from the hydroxyl, C₁-C₂ alkoxy, amino and C₁-C₂ (di)alkylamino radicals.

20. Composition according to any one of Claims 1 to 19, in which the dye of formula (I) is chosen from

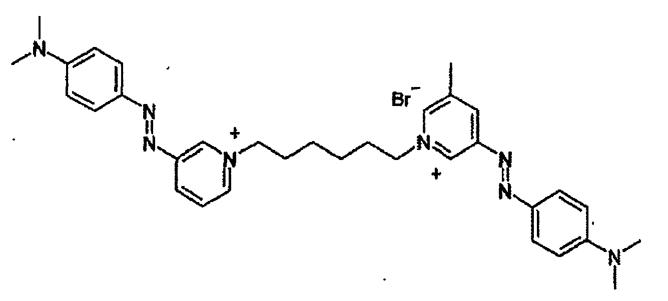

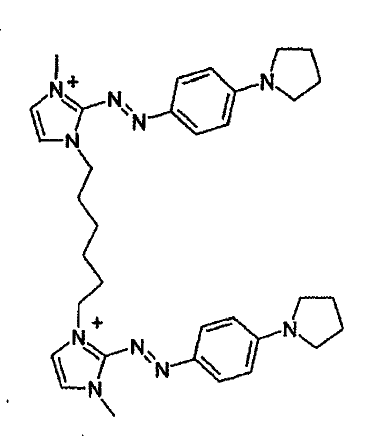

21. Composition according to any one of Claims 1 to 19, in which the dye is

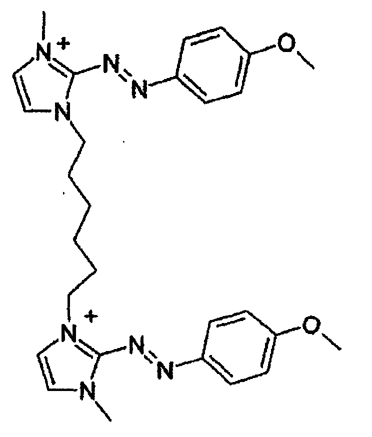

22. Composition according to any one of Claims 1 to 21, furthermore comprising an oxidation base.

23. Composition according to Claim 22, in which the oxidation base is chosen from paraphenylenediamines, bisphenylalkylenediamines, paraaminophenols, ortho-aminophenols, heterocyclic bases and their addition salts with an acid.

24. Composition according to either of Claims 22 and 23, in which the oxidation base(s) are present in a quantity between 0.001 and 10%, preferably between 0.005 and 6%.

25. Composition according to any one of Claims 1 to 24, comprising at least one coupler.

26. Composition according to Claim 25, in which the coupler is chosen from metaphenylenediamines, meta-aminophenols, meta-diphenols, naphthalene couplers and heterocyclic couplers and their addition salts with an acid.

27. Composition according to any one of Claims 1 to 26, furthermore comprising an oxidizing agent, preferably hydrogen peroxide.

28. Process for the oxidation dyeing of keratinous fibers and in particular of human keratinous fibers such as the hair, **characterized in that** at least one dyeing composition as defined in any one of Claims 1 to 26 is applied to the fibers.

29. Process according to Claim 28, in which the dyeing composition contains an oxidizing agent.

30. Process according to Claim 29, in which the oxidizing agent is mixed at the time of use with the dyeing composition.

31. Process according to either of Claims 29 and 30, in which the oxidizing agent is applied to the fibers in the form of an oxidizing composition simultaneously with or sequentially to the dyeing composition.

32. Process for the oxidation dyeing of human keratinous fibers, **characterized in that** at least one dyeing composition as defined in any one of Claims 1 to 21, furthermore comprising at least one oxidation base and optionally at least one coupler, in the presence of an oxidizing agent, is applied to the fibers.

33. Process according to Claim 32, in which the oxidizing agent is mixed at the time of use with the dyeing composition.

34. Process according to Claim 32, in which the oxidizing agent is applied to the fibers in the form of a dyeing composition simultaneously with or sequentially to the dyeing composition.

35. Multi-compartment device or multi-compartment dyeing "kit", in which a first compartment comprises a composition as defined in any one of Claims 1 to 26 and a second compartment comprises an oxidizing composition.

## Patentansprüche

1. Zusammensetzung zum Färben von menschlichen Keratinfasern, die mindestens einen dikationischen Diazofarbstoff der folgenden Formel (I) enthält: 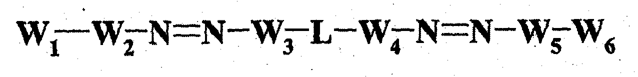 in der Formel:
- W₁ und W₆ bedeuten unabhängig voneinander eine Gruppe NR₁R₂ oder OR,
- W₂ und W₅ bedeuten unabhängig voneinander eine aromatische Gruppe auf Kohlenstoffbasis der folgenden Formel (II): 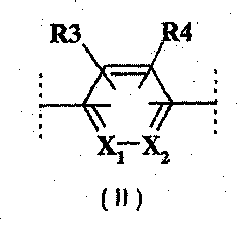
- W₃ und W₄ bedeuten unabhängig voneinander eine heteroaromatische Gruppe der folgenden Formeln (III) und (IV): 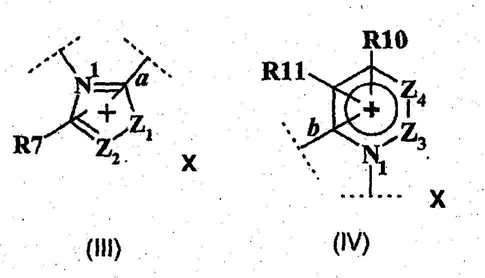 in den Formeln:
- X₁ bedeutet eine Gruppe CR₅,
- X₂ bedeutet eine Gruppe CR₆,
- Z₁ bedeutet eine Gruppe NR₈,
- Z₂ bedeutet ein Stickstoffatom oder eine Gruppe CR₉,
- Z₃ bedeutet eine Gruppe CR₁₂,
- Z4 bedeutet eine Gruppe CR₁₃,
- N¹ des 5-gliedrigen Rings der Formel (III) ist an die Gruppe L gebunden und die Bindung a dieses 5-gliedrigen Rings geht zur Azogruppe der Formel (I),
- die Bindung b des 6-gliedrigen Rings der Formel (IV) geht zur Azogruppe der Formel (I) und N¹ des 6-gliedrigen Rings der Formel (IV) ist an die Gruppe L gebunden,
- die Gruppen R_{1,} R_{2,} R_{3,} R_{4,} R_{5,} R_{6,} R_{7,} R_{9,} R_{10,} R_{11,} R₁₂ und R₁₃ bedeuten gemeinsam oder unabhängig voneinander eine geradkettige oder verzweigte C₁₋₁₆-Kohlenwasserstoffgruppe, die einen oder mehrere 3- bis 6-gliedrige Kohlenstoffringe bilden kann, wobei sie gesättigt oder ungesättigt vorliegen können, wobei ein oder mehrere Kohlenstoffatome der Kohlenstoffkette durch ein Sauerstoffatom, Stickstoffatom oder Schwefelatom oder durch eine Gruppe SO₂ ersetzt sein können, und bei der die Kohlenstoffatome unabhängig voneinander mit einem oder mehreren Halogenatomen substituiert sein können; die Gruppen R₁, R₂, R₃, R₄, R₅, R₆, R₇, R_{9,} R_{10,} R_{11,} R₁₂ und R₁₃ können Wasserstoff bedeuten; wobei die Gruppen R_{1,} R_{2,} R_{3,} R_{4,} R_{5,} R_{6,} R_{7,} R_{9,} R_{10,} R_{11,} R₁₂ und R₁₃ weder eine Peroxybindung noch eine Diazogruppe oder Nitrosogruppe enthalten, und L eine zweiwertige Gruppe ist, die unter den geradkettigen oder verzweigten C₁₋₈-Alkylgruppen ausgewählt ist, die gegebenenfalls mit einer oder mehreren Gruppen substituiert sind, die unter Hydroxy, Alkoxy(C₁₋₂), (Poly)hydroxyalkoxy(C₂₋₄), Amino, (Di)alkylamino(C₁₋₂), Carboxy oder Sulfo ausgewählt sind, und vorzugsweise eine geradkettige oder verzweigte C₁₋₈-Alkylgruppe ist,
- die Gruppe R₈ bedeutet eine geradkettige oder verzweigte C₁₋₈-Alkylgruppe, die gegebenenfalls mit einer oder mehreren Gruppen substituiert ist, die unter Hydroxy, Alkoxy(C₁₋₂), (Poly)hydroxyalkoxy(C₂₋₄), Amino, (Di)alkylamino(C₁₋₂), Carboxy, Sulfo ausgewählt sind; eine gegebenenfalls substituierte Phenylgruppe,
- R₇ kann mit R₉, R₁₀ kann mit R₁₁ und R₁₂ kann mit R₁₃ einen aromatischen Kohlenstoffring bilden,
- R ist eine C₁₋₃-Alkylgruppe,
- X bedeutet ein organisches oder anorganisches Anion.

2. Zusammensetzung nach Anspruch 1, wobei R₁ und R₂ bedeuten: ein Wasserstoffatom; eine geradkettige oder verzweigte C₁₋₆-Alkylgruppe, die gegebenenfalls mit einer oder mehreren Gruppen substituiert ist, die unter Hydroxy, Alkoxy(C₁₋₂), (Poly)hydroxyalkoxy(C₂₋₄), Amino, (Di)alkylamino(C₁₋₂), Carboxy oder Sulfo ausgewählt sind; eine Phenylgruppe, die gegebenenfalls mit einer oder mehreren Gruppen substituiert ist, die ausgewählt sind unter Hydroxy, Alkoxy(C₁₋₂), (Poly)hydroxyalkoxy(C₂₋₄), Amino, (Di)alkylamino(C₁₋₂), Carboxy, Sulfo oder einem Halogenatom; eine Alkylsulfonyl(C₁₋₄)gruppe; eine Arylsulfonylgruppe; eine Gruppe, die unter Pyrazolyl, Imidazolyl, Thiazolyl, Oxazolyl, Thiadiazolyl, Triazolyl, Pyrrolyl, Pyridyl, Pyrimidinyl, Triazinyl, Pyrazinyl, Pyridazinyl ausgewählt ist; R₁ und R₂ bilden gemeinsam einen 5-, 6-, 7- oder 8-gliedrigen Heterocyclus, der gegebenenfalls substituiert ist mit einer oder mehreren Gruppen Hydroxy; Amino; -NR₁₄R₁₅, wobei R₁₄ und R₁₅ unabhängig voneinander ein Wasserstoffatom, eine geradkettige oder verzweigte C₁₋₄-Alkylgruppe bedeuten, die gegebenenfalls mit einer oder mehreren Gruppen substituiert ist, die unter einem Halogenatom, einer Hydroxygruppe, einer Alkoxy(C₁₋₂)gruppe, einer Aminogruppe, einer Amino(di)alkylgruppe mit C₁-C₂, einer (Poly)hydroxyalkylaminogruppe ausgewählt sind; Sulfonylamino; Carboxy; Carboxamido; C₁₋₆-Alkyl, wobei diese Gruppe gegebenenfalls substituiert ist mit einer oder mehreren Hydroxygruppen; Amino;-NR₁₄R₁₅; R₁ und R₂ bilden gemeinsam eine 5- oder 6-gliedrige heteroaromatische Gruppe.

3. Zusammensetzung nach Anspruch 2, wobei R₁ und R₂ bedeuten: ein Wasserstoffatom; eine geradkettige oder verzweigte C₁₋₃-Alkylgruppe, die gegebenenfalls mit einer oder mehreren Gruppen substituiert ist, die unter Hydroxy, Alkoxy(C₁₋₂), Amino, (Di)alkylamino(C₁₋₂), Carboxy oder Sulfo ausgewählt sind; eine Phenylgruppe, die gegebenenfalls mit einer oder mehreren Gruppen substituiert ist, die unter Amino, (Di)alkylamino(C₁₋₂) oder (Poly)hydroxyalkylamino ausgewählt sind; R₁ und R₂ bilden gemeinsam einen 5- oder 6-gliedrigen Heterocyclus, der gegebenenfalls substituiert ist mit einer oder mehreren Gruppen, die ausgewählt sind unter Methyl, Hydroxy; Amino; (Di)methylamino; Sulfonylamino; (Poly)hydroxyalkylamino(C₂₋₄), Carboxy; Carboxamido.

4. Zusammensetzung nach Anspruch 3, wobei R₁ und R₂ bedeuten: ein Wasserstoffatom; Methyl, Ethyl, 2-hydroxymethyl, 2-Aminoethyl; 1-Carboxymethyl, 2-Carboxyethyl, 2-Sulfonylethyl, 2-Methoxyethyl; eine Phenylgruppe, die gegebenenfalls mit einer oder mehreren Gruppen substituiert ist, die unter Amino, (Di)alkylamino(C₁₋₂) oder (Poly)hydroxyalkylamino ausgewählt sind; R₁ und R₂ bilden gemeinsam eine 5- oder 6-gliedrige heterocyclische Pyrrolidin- oder Piperidingruppe, die gegebenenfalls mit einer oder mehreren Gruppen Methyl, Hydroxy, Amino, (Di)methylamino, Carboxy, Carboxamido substituiert ist.

5. Zusammensetzung nach Anspruch 4, wobei R₁ und R₂ bedeuten: ein Wasserstoffatom; Methyl, 2-Hydroxyethyl; eine Phenylgruppe, die gegebenenfalls substituiert ist mit einer Gruppe Amino, (Di)methylamino, (Di)(2-hydroxyethyl)amino; R₁ und R₂ bilden Pyrrolidin, 3-Hydroxypyrrolidin, Piperidin, 3-Hydroxypiperidin; R₁ und R₂ bilden gemeinsam Pyrazol, Imidazol, Pyrrol, Triazol, Pyridazin, Pyrimidin.

6. Zusammensetzung nach einem der Ansprüche 1 bis 5, wobei R₃, R_{4,} R_{5,} R_{6,} R_{10,} R_{11,} R₁₂ und R₁₃ bedeuten: ein Wasserstoffatom; eine geradkettige oder verzweigte C₁₋₄-Alkylgruppe, die gegebenenfalls mit einer oder mehreren Gruppen substituiert ist, die unter Hydroxy, Alkoxy(C₁₋₂), (Poly)hydroxyalkoxy(C₂₋₄), Amino, (Di)alkylamino(C₁₋₂), Carboxy oder Sulfo ausgewählt sind; eine Phenylgruppe, die gegebenenfalls mit einer oder mehreren Gruppen substituiert ist, die ausgewählt sind unter Hydroxy, Alkoxy(C₁₋₂), (Poly)hydroxyalkoxy(C₂₋₄), Amino, (Di)alkylamino(C₁₋₂), Carboxy, Sulfo oder einem Halogenatom; Carboxy; Sulfonylamino; Sulfo; Alkoxy(C₁₋₂); (Poly)hydroxyalkoxy(C₂₋₄); Amino; (Di)alkylamino(C₁₋₂); (Poly)hydroxyalkylamino(C₂₋₄).

7. Zusammensetzung nach Anspruch 6, wobei die Gruppen R_{3,} R_{4,} R_{5,} R_{6,} R_{10,} R_{11,} R₁₂ und R₁₃ bedeuten: ein Wasserstoffatom; eine C₁₋₄-Alkylgruppe, die gegebenenfalls mit einer oder mehreren Gruppen substituiert ist, die ausgewählt sind unter Hydroxy, Amino oder (Di)alkylamino(C₁₋₂); Carboxy; Alkoxy(C₁₋₂); Amino; (Di)alkylamino(C₁₋₂); (Poly)hydroxyalkylamino(C₂₋₄).

8. Zusammensetzung nach Anspruch 7, wobei die Gruppen R₃, R₄, R_{5,} R_{6,} R_{10,} R_{11,} R₁₂ und R₁₃ bedeuten: ein Wasserstoffatom; Methyl, Phenyl, 2-Hydroxymethyl, Carboxy, Methoxy, Ethoxy, 2-Hydroxyethyloxy, Amino, Methylamino, Dimethylamino, 2-Hydroxyethylamino.

9. Zusammensetzung nach einem der Ansprüche 1 bis 8, wobei die Gruppen R₇ und R₉ bedeuten: ein Wasserstoffatom; eine geradkettige oder verzweigte C₁₋₄-Alkylgruppe, die gegebenenfalls mit einer oder mehreren Gruppen substituiert ist, die ausgewählt sind unter Hydroxy, Alkoxy(C₁₋₂), (Poly)hydroxyalkoxy(C₂₋₄), Amino, (Di)alkylamino(C₁₋₂), Carboxy oder Sulfo; eine gegebenenfalls substituierte Phenylgruppe; eine Carboxygruppe; eine Sulfonylaminogruppe.

10. Zusammensetzung nach Anspruch 9, wobei die Gruppen R₇ und R₉ bedeuten: ein Wasserstoffatom; eine Phenylgruppe, eine C₁₋₄-Alkylgruppe, die gegebenenfalls mit einer oder mehreren Gruppen substituiert ist, die ausgewählt sind unter Hydroxy, Amino, (Di)alkylamino(C₁₋₂), Carboxy.

11. Zusammensetzung nach Anspruch 9, wobei die Gruppen R₇ und R₉ bedeuten: ein Wasserstoffatom, Methyl, Phenyl, 2-Hydroxymethyl, Carboxy.

12. Zusammensetzung nach einem der Ansprüche 1 bis 11, wobei die Gruppe R₈ bedeutet: eine C₁₋₄-Alkylgruppe, die gegebenenfalls mit einer oder mehreren Gruppen substituiert ist, die ausgewählt sind unter Hydroxy, Alkoxy(C₁₋₂), Amino, (Di)alkylamino(C₁₋₂), Carboxy, Sulfo.

13. Zusammensetzung nach Anspruch 12, wobei die R₈ bedeutet:
Methyl, Ethyl, 2-Hydroxyethyl, 1-Carboxymethyl, 2-Carboxyethyl, 2-Sulfonylethyl.

14. Zusammensetzung nach einem der Ansprüche 1 bis 13, wobei W und W6 NR1R2 bedeuten und R1 und R2 unter Wasserstoff und einer Alkylgruppe ausgewählt sind, die mit Hydroxy oder Amino substituiert sein kann.

15. Zusammensetzung nach einem der Ansprüche 1 bis 13, wobei W1 und W6 OR bedeuten und R eine Methylgruppe ist.

16. Zusammensetzung nach Anspruch 1, wobei W2 und W5 bedeuten: eine Phenylgruppe, die gegebenenfalls substituiert sein kann mit Alkyl, Alkoxy, Amino, einer mono- oder disubstituierten Aminogruppe; Methoxy; Methyl, Amino, Methylamino, Dimethylamino.

17. Zusammensetzung nach einem der vorhergehenden Ansprüche, wobei die Gruppen W3 und W4 unter den Gruppen Imidazolinium, Triazolium, Pyridinium ausgewählt sind.

18. Zusammensetzung nach einem der vorhergehenden Ansprüche, wobei der Farbstoff der Formel (I) so vorliegt, dass
- W2 und W5 Phenylengruppen sind,
- W3 und W4 Imidazoliniumgruppen sind.

19. Zusammensetzung nach einem der Ansprüche 1 bis 18, wobei L eine geradkettige oder verzweigte C₁₋₈-Alkylengruppe bedeutet, die gegebenenfalls mit einer oder mehreren Gruppen substituiert ist, die ausgewählt sind unter Hydroxy, Alkoxy(C₁₋₂), Amino, (Di)alkylamino(C₁₋₂).

20. Zusammensetzung nach einem der Ansprüche 1 bis 19, wobei der Farbstoff der Formel (I) ausgewählt ist unter:

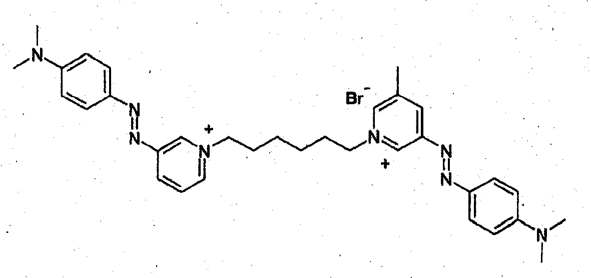

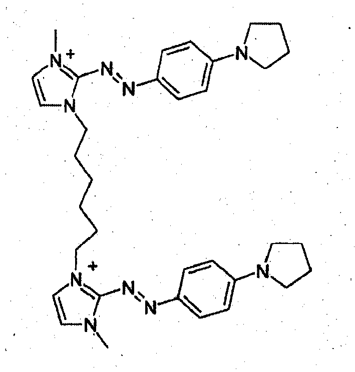

21. Zusammensetzung nach einem der Ansprüche 1 bis 19, wobei der Farbstoff ist:

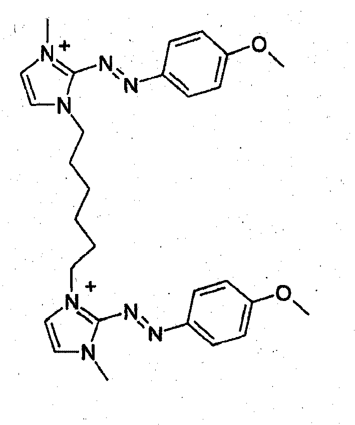

22. Zusammensetzung nach einem der Ansprüche 1 bis 21, die ferner eine Oxidationsbase enthält.

23. Zusammensetzung nach Anspruch 22, wobei die Oxidationsbase unter den *p*-Phenylendiaminen, Bisphenylalkylendiaminen, p-Aminophenolen, o-Aminophenolen, heterocyclischen Basen und deren Additionssalzen mit einer Säure ausgewählt ist.

24. Zusammensetzung nach einem der Ansprüche 22 oder 23, wobei die Oxidationsbase oder die Oxidationsbasen in einem Mengenanteil von 0,001 bis 10 % und vorzugsweise 0,005 bis 6 % enthalten sind.

25. Zusammensetzung nach einem der Ansprüche 1 bis 24, die mindestens einen Kuppler enthält.

26. Zusammensetzung nach Anspruch 25, wobei der Kuppler unter den *m*-Phenylendiaminen, *m*-Aminophenolen, *m*-Dihydroxybenzolen, Naphthalinkupplern und heterocyclischen Kupplern und deren Additionssalzen mit einer Säure ausgewählt ist.

27. Zusammensetzung nach einem der Ansprüche 1 bis 26, die ferner ein Oxidationsmittel und vorzugsweise Wasserstoffperoxid enthält.

28. Verfahren zum oxidativen Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 26 aufgebracht wird.

29. Verfahren nach Anspruch 28, wobei die Farbmittelzusammensetzung ein Oxidationsmittel enthält.

30. Verfahren nach Anspruch 29, wobei das Oxidationsmittel bei der Anwendung mit der Farbmittelzusammensetzung vermischt wird.

31. Verfahren nach einem der Ansprüche 29 oder 30, wobei das Oxidationsmittel in Form einer oxidierenden Zusammensetzung gleichzeitig mit oder nach der Farbmittelzusammensetzung auf die Fasern aufgebracht wird.

32. Verfahren für die oxidative Färbung von menschlichen Keratinfasern, **dadurch gekennzeichnet, dass** mindestens eine Farbmittelzusammensetzung nach einem der Ansprüche 1 bis 21, die ferner mindestens eine Oxidationsbase und gegebenenfalls mindestens einen Kuppler enthält, in Gegenwart eines Oxidationsmittels auf die Fasern aufgebracht wird.

33. Verfahren nach Anspruch 32, wobei das Oxidationsmittel bei der Anwendung mit der Farbmittelzusammensetzung vermischt wird.

34. Verfahren nach Anspruch 32, wobei das Oxidationsmittel in Form einer oxidierenden Zusammensetzung gleichzeitig mit oder nach der Farbmittelzusammensetzung auf die Fasern aufgetragen wird.

35. Vorrichtung mit mehreren Abteilungen oder "Kit" zum Färben mit mehreren Abteilungen, bei der eine erste Abteilung eine Zusammensetzung, wie sie in einem der Ansprüche 1 bis 26 definiert ist, und eine zweite Abteilung eine oxidierende Zusammensetzung enthält.
